# EUROPEAN PATENT SPECIFICATION

(11) **EP 0 573 457 B1**
(45) Date of publication and mention of the grant of the patent: **10.07.1996**
(21) Application number: 92904574.8
(22) Date of filing: 10.02.1992
(51) Int. Cl.: A61M 11/06, B05B 7/00

(54) **NEBULIZER**
Inhalator
NEBULISEUR

(30) Priority: 28.02.1991 GB 9104199
(43) Date of publication of application: 15.12.1993
(73) Proprietor: INTERSURGICAL LIMITED, Twickenham, Middlesex TW2 6RS (GB)
(72) Inventor: BELLM, Howard, Geoffrey, Berkshire (GB)
(74) Representative: Wright, Hugh Ronald
(86) International application number: GB9200239
(87) International publication number: WO9215354

(56) References cited:
- EP-A- 0 276 939
- DE-C- 3 513 876
- US-A- 4 344 574
- US-A- 4 560 519
- US-A- 4 588 129
- US-A- 4 746 067

## Description

The present invention relates to nebulizers. Nebulizers are used to produce a liquid in fine droplet form in a gas flow, typically a liquid in the form of medication which is to be entrained in an air or oxygen gas flow and which is then breathed by a patient whereby the medication is applied to the patient through the lungs.

Nebulizers have become widely used in hospitals and other medical situations.

Such nebulizers have a particular application in delivery of drugs for the treatment of lung diseases. Thus the drug may be given as an inhaled drug aerosol, and this has many advantages over drugs which are taken systemically. Thus the onset of the drug action is quicker, the therapeutic dose is lower, which reduces the side effects, and the drug delivery may be more selective.

It has become more widely accepted that different clinical needs require the nebulized drug to be targeted to specific areas of the lungs. Many factors can affect where the aerosol is deposited and the depth of penetration, including the patient's condition and breathing pattern. However, research has clearly shown that aerosol droplet size plays an important role in the area of deposition, that is the part of the lung in which the drug is deposited. To ensure that the drug is delivered to the correct part of the lung the correct droplet size must be provided.

The applicants have tested many widely available nebulizers and find that as a general rule, they provide a mass median diameter (MMD) which prevents the drug or, at least, much of the drug passing through to the alveoli.

As an example of the use of this aspect of the invention, the ability to deliver a drug to the alveoli of the lung can be particularly important in treating diseases of the lungs in immuno compromised patients with pneumocystis pneumonia. The *pneumocystis carinii* protozoa can be treated by administering Pentamidine, but a particular difficulty is the toxicity of the drug. If one were able to administer Pentamidine in aerosol form of such a droplet size that the droplets were able to pass through directly to the alveoli and there be deposited, then the quantity of drug applied could be reduced considerably and this would reduce the side effects of the drug, whilst improving its efficacy.

It is therefore desirable to provide a nebulizer which produces the desired droplet sizes, in the range 0.5 - 5 micron, preferably 0.5 - 2 micron for depositing in the alveoli or 2 - 5 micron for depositing in the tracheobronchial area of the lung.

EP-A-0 276 939 shows a nebulizer of the general type set out in the preamble of claim 1.

The present invention provides a nebulizer comprising:
a base part including a base wall extending to an apex;
a gas input including an input connector;
a gas jet hole extending through the base wall connecting with the interior of the input connector;
a gas/liquid output;
a flange plate extending above said base wall over the gas jet hole, the under surface of said flange plate generally matching the upper surface of the base wall and being spaced from said base wall by a sufficiently small distance to provide a capillary path;
a reservoir for liquid being provided by said base wall communicating with said capillary path; and,
a gas/liquid mixing jet for mixing gas from the gas jet hole through the base wall with liquid from the capillary path, the gas/liquid mixing jet comprising a hole through the flange plate opposite and in axial alignment with said gas jet hole through the base wall;
an annular baffle plate, including a target centrally mounted on said annular baffle plate opposite said gas/liquid mixing jet against which the gas/liquid jet formed by the gas/liquid mixing jet impinges to nebulize the liquid;
and,
outlet aperture means adjacent the outer peripheral edge of said baffle plate and connecting with the gas/liquid output for allowing direct passage of the nebulized gas/liquid mixture from the nebulizer characterised by a ring member, in use, positioned so as to extend from the surface of the annular baffle plate, and being generally coaxial with the target, and in that, with the nebulizer in an upright orientation, the lowermost edge of the ring member extends below the lowermost point of the target.

Preferably the target is semispherical.

Preferably the radius of the target is 2 - 5mm, preferably substantially 3.5mm.

Preferably the diameter of the ring means is 15 - 25mm, preferably approximately 20mm diameter.

Preferably the depth of the cross section of the ring means is 1 - 5mm, preferably 1.5mm.

Preferably the depth of the cross section of the ring means is approximately 3mm.

Preferably the target comprises a conical member.

Preferably the angle of the surface of the conical target with respect to its axis is between 55 degrees and 80 degrees.

Preferably the angle of the surface of the conical target with respect to its axis is approximately 70 degrees.

Preferably the ring means is mounted by leg means, to the flange plate.

Preferably said nebulizer provides droplets of a mass median diameter (MMD) of less than 3.5 micron.

Preferably the droplets have a mass median diameter (MMD) of less than approximately 1.5 micron.

Nebulizers incorporating the various aspects of the invention will now be described by way of example only and with reference to the accompanying drawings in which:-
Figure 1 is an axial section of a known nebulizer,
Figure 2 is an axial section of a first nebulizer according to the present invention in a vertical orientation,
Figure 3 is an axial section of the first nebulizer of Figure 2 in a horizontal orientation,
Figure 4 is a perspective view of the first nebulizer of Figure 2,
Figures 5 and 6 are side views of two of the three components forming the first nebulizer of Figure 2,
Figure 7 is a perspective view of a third part of the first nebulizer of Figure 2,
Figure 8 is an axial section of a second nebulizer according to the present invention in a vertical orientation,
Figure 9 is an axial section of a third nebulizer according to the present invention in a vertical orientation,
Figure 10 is a graph of the droplet size distribution of a nebulizer of the type shown in Figure 1,
Figure 11 is a graph of the droplet size distribution of a nebulizer in accordance with U.S. Patent No.4,588,129.
Figure 12 is a graph of the droplet size distribution of a nebulizer in accordance with U.S. Patent No.4,746,067,
Figure 13 is a graph of the droplet size distribution of a nebulizer according to our European Patent Application 88300391.5,
Figure 14 is a graph of the droplet size distribution of a nebulizer in accordance with Figure 2,
Figure 15 is a graph of the droplet size distribution of a nebulizer in accordance with Figure 8,
Figure 16 is a graph of the droplet size distribution of a nebulizer in accordance with Figure 2 but modified similar to that of U.S. Patent No.4,588,129, that is having a rearwardly extending chamber between the target and ring.
Figure 17 is a graph of the droplet size distribution of a nebulizer according to Figure 9.
Figure 18 shows a diagrammatic view of the nebulized jet of gas/liquid and the path of said gas/liquid jet.

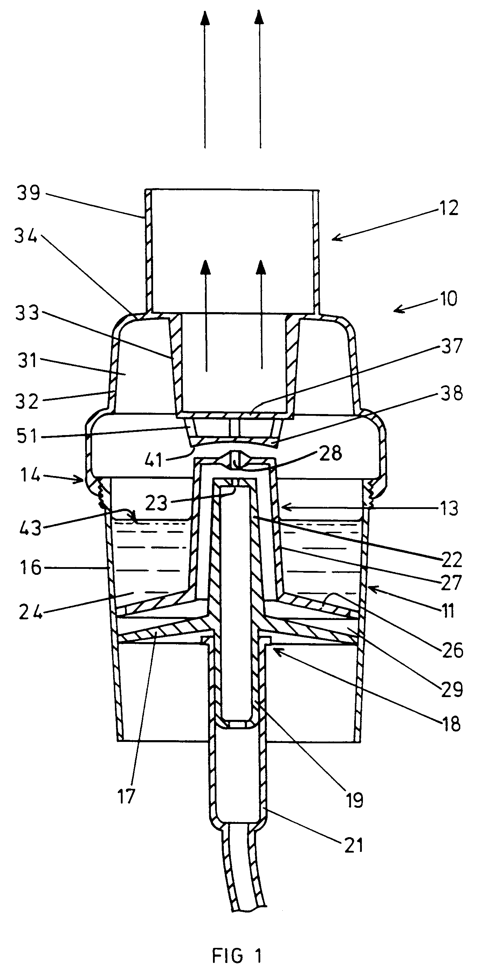

Referring now to Figure 1 there is shown a known nebulizer 10 moulded of rigid plastics material in three separable parts, as base part 11, a cap part 12 and a flange plate 13. The base part 11 is screw threadedly engaged with the cap part 12 by means of a continuous screw thread at 14 (not shown in detail).

The base part 11 comprises a generally cylindrical outer wall 16, a transverse wall 17 extending across the lower part of the cylindrical outer wall 16 with a slight upward slope to its centre so that it comprises a shallow conical wall. Passing through the axial centre of the transverse wall 17 is the gas inlet 18, the gas inlet being defined by a gas inlet connector 19 extending downwardly from the transverse wall 17 to which a standard type gas supply tube 21 may be connected. The gas inlet 18 also extends upwardly from the transverse wall 17 by means of the extending tube portion 22, the upper end of the tube portion 22 being generally in the plane of the upper end of the cylindrical outer wall 16 and being closed by an end wall which includes an aperture therein forming a gas jet 23. It will be understood that the volume between the inner surface of the cylindrical outer wall 16 and the outer surface of the tube portion 22 forms a reservoir 24 for liquid.

The flange plate 13 comprises a shallow conical skirt portion 26 and an upright tubular part 27 extending from the skirt 26, the upper end of the tubular part 27 being closed, but including a gas/liquid mixing jet 28.

In use the configuration is such that a very narrow gap (forming a capillary path 29) is formed between the undersurface of the skirt part 26 and the upper surface of the transverse wall 17 and between the inner wall of the tubular part 27 and the outer wall of the tube portion 22 whereby liquid in the reservoir 24 may be drawn up through this capillary path 29 from the circular outer lip of the skirt part 26 within the reservoir up to the gas/liquid jet 28.

The cap part 12 comprises a screw threaded portion to engage with the base part 11, and a substantially closed cylindrical upper portion which forms a reservoir 31 for the liquid within the reservoir 24 when the nebulizer 10 is disposed horizontally. The reservoir 31 is provided between the outer cylindrical wall 32 and an inner cylindrical wall 33, the two cylindrical walls 32 33 being joined at their outer end by a generally transverse annular wall 34, an upstanding cylindrical portion extending from this annular wall 34 to provide an outlet connector for the gas/liquid mixture. A transverse wall 37 closes the inner end of the inner cylindrical wall 33 and mounts a baffle plate 38 which is disposed just above the gas/liquid mixing jet 28. The undersurface of the baffle plate 38 includes a shaped semi-cylindrical target 41 aligned with the gas/liquid mixing jet 28. Aperture means (not shown) is provided to allow the nebulized gas/liquid mixture to pass from within the nebulizer to the output connector 39, which may be provided by apertures in the diaphragm wall 37.

In operation, the cap part 12 is removed from the base part 11 and the necessary liquid to be nebulized is poured into the reservoir 24. The cap part 12 is then re-engaged with the base part 11 and the gas supply tube 21 connected to the gas inlet connector 19.

Gas may then be passed under pressure through to the gas nozzle 23 and the action of this causes liquid to be drawn up through the capillary path 29 and to be mixed with the gas and nebulized at the gas/liquid mixing jet 28. By careful design of the jet sizes and relationship with the baffle plate 38 a nebulized mixture of gas and liquid is ejected from the region of the gas/liquid mixing jet 28 and passes out to the output connector 39.

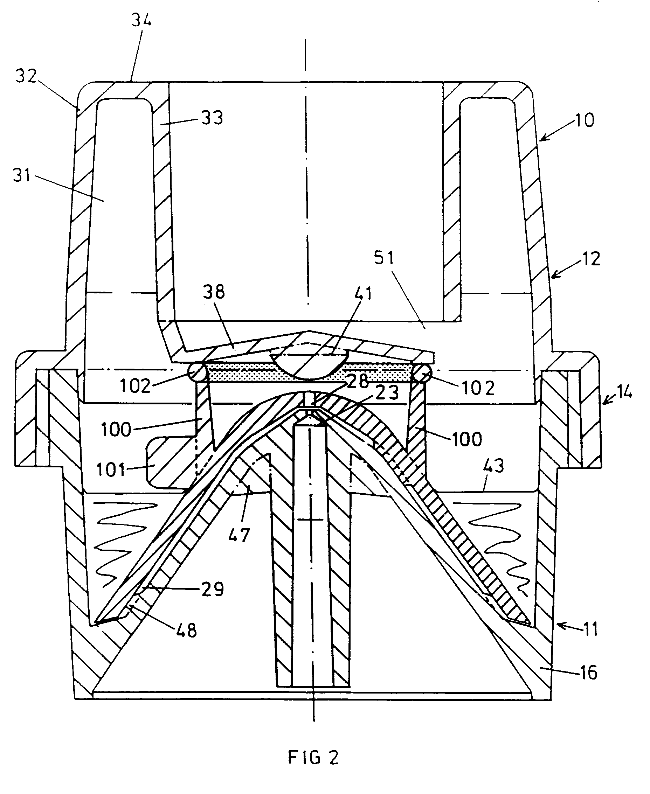

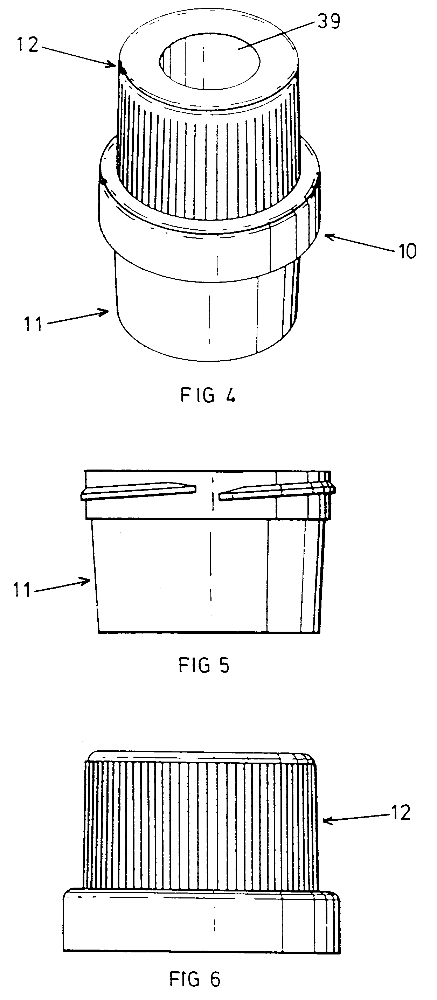

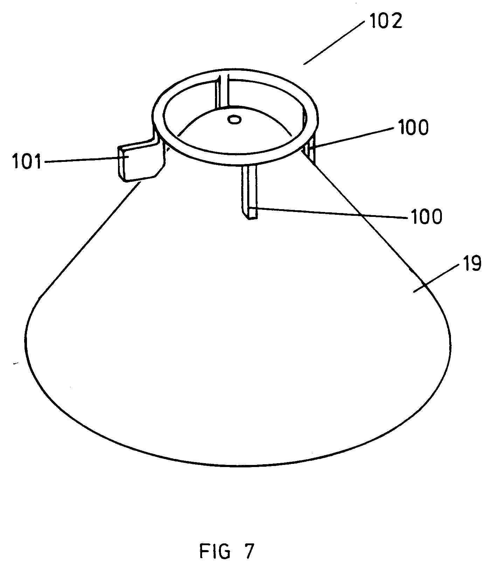

A first nebulizer according to the invention is illustrated in Figures 2 - 7. Similar parts are indicated with the same reference numerals and the general arrangement will not be described further, as it is generally similar to the nebulizer of Figure 1.

In general terms it will be seen that the first nebulizer of the invention is considerably shorter in axial length than the nebulizer of Figure 1 thereby making it more compact, the reservoir 31 is somewhat wider which makes it easier to fill. Other advantages will become apparent.

In the base part 11 of the nebulizer according to the invention the generally transverse wall 17 and tube portion 22 is replaced by a wall 46 which is more steeply conical than the transverse wall 17 of Figure 1. Thus, the outer periphery of the closure wall 46 is disposed generally adjacent to the base of the cylindrical wall 16 and extends upwardly at an acute angle between 20° and 60°, preferably 45°, although with a curved central part. The gas inlet connector 19 is mounted directly in this closure wall 46 on the axis of the nebulizer. The gas inlet connector 19 extends downwardly from this central region of the closure wall 46 to a point just above the lower edge of the nebulizer. There are also provided strengthening flanges 47 which extend between the upper end of the gas inlet connector 19 and the under side of the closure wall 46 adjacent the central area.

Similarly, the flange plate 13 is shaped so as to follow the contours of the upper surface of the closure wall 46 and thereby comprises a generally conical shape, the shape of the under surface of the flange plate 13 closely matching the upper surface of the closure wall 46 to provide therebetween capillary path 29. Spacing between the cap part 12 and closure wall 46 is maintained by spacers moulded to the top surface of the closure wall 46 and the under surface of flange plate 13. The gas/liquid mixing jet 28 comprises a jet passing through the axis of the flange plate 13.

Because the shape of the reservoir 24 has been changed by means of the conical lower wall of the reservoir, to maintain the same volume, the outer diameter of the cylindrical outer wall 16 must be increased from 35mm (in the configuration of Figure 1) to 42mm.

This increase in diameter also allows changes in the cap part 12. As will be understood from Figure 1, in order to connect with standard tubing the output connector 39 has specific dimensions and in particular, has an inner diameter of 22mm with a specific taper. Because of the increase in diameter of the nebulizer it is now possible to arrange for the inner cylindrical wall 33 to be of 22mm diameter and this means that this inner cylindrical wall 33 can itself form the output nozzle 39.

The baffle plate 38 and target 41 are combined with the wall 33, the target 41 comprising a divergent surface diverging away from the jet 28. The target 41 comprises a semi spherical protrusion from the underside of the wall 38. Thus the distance between the target 41 and the top surface of the flange plate 13 rapidly diverged from one another within increasing distance from the jet. It has been found that the particular arrangement of surfaces creates a very good nebulizer.

Apertures 51 are provided around the inner end of the inner cylindrical wall 33 to allow the nebulized gas liquid mixture to pass out of the interior of the nebulizer to the output connector 39. To prevent the cylindrical connector portion of the tube engaging with the output connector 39 from covering the apertures 51, the apertures 51 are provided in an inwardly extending conical inner part 52 of the cylindrical wall 33.

Instead of continuous threads being provided on the threaded portions of the base part 11 and cap part 12 there are provided interrupted thread portions which require less turning to tighten than a complete screw thread.

It will be understood that all parts of the nebulizer are moulded from the same rigid plastic which may be rigid polystyrene (styrene butadiene block copolymer).

Four legs 100 extend from the upper surface of flange plate 13, one of which carries a fin 101 for ease of handling, and a ring 102 joins the upper end of each leg 100. These parts may all be arranged so as to be moulded with the flange plate 13.

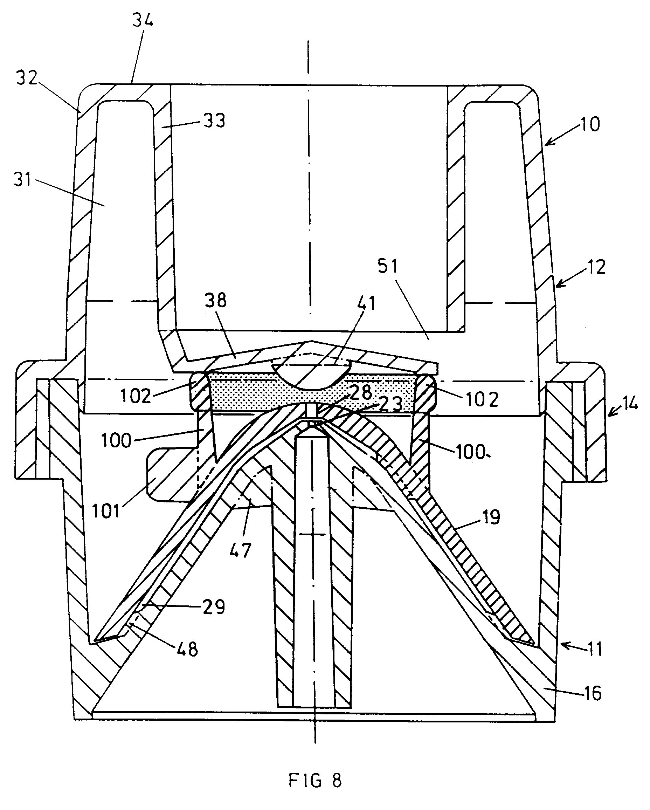

As can be seen from Figure 8, the uppermost surface of the ring 102 abuts the undersurface of the baffle plate 38.

The ring 102 is of an overall diameter such that it extends substantially just inside the peripheral edge of the baffle plate 38 (the outer diameter of the baffle plate 38) being 22mm and the diameter of the ring (overall maximum diameter) being 20mm. In cross section, the ring is 1.6mm wide and 1.5mm deep.

The semispherical target 41 has a radius of 3.67mm, and the gap between the top surface of the gas/liquid mixing jet 28 and the closest point of the surface of the target 41 is 1mm. The other dimensions are as shown on Figure 2. It will be noted from Figure 2 that the lower edge of the ring 102 is below the lower most point of the target 41.

Figure 2 shows the nebulizer according to the invention in the vertical orientation and the level of liquid within the reservoir 24 is illustrated at 43. It can be seen that this liquid level is below the gas/liquid mixing jet 28.

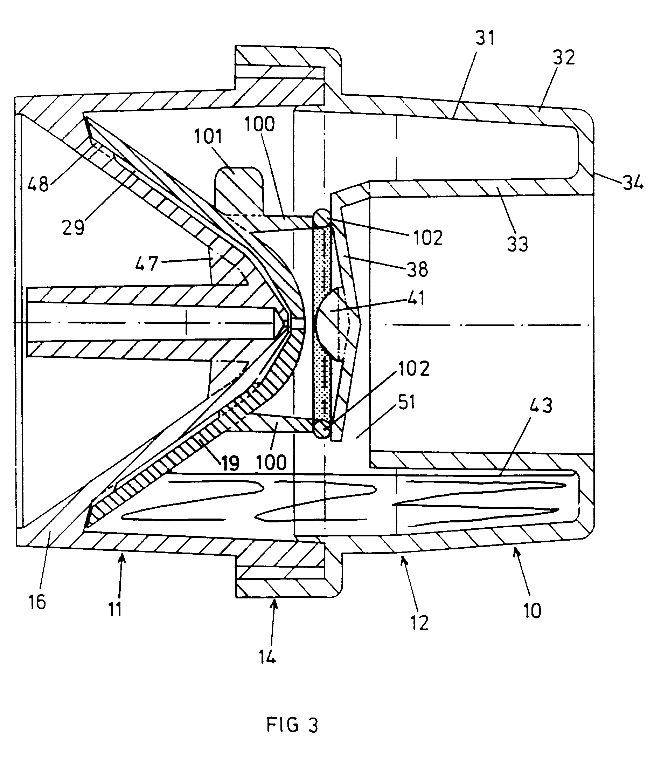

Figure 3 shows the nebulizer when in the horizontal orientation from which it will be seen that the level of the liquid 43 (which is now distributed between the reservoir 24 and the reservoir 31 is still below the gas/liquid mixing jet 28 and the apertures 51 so that no spillage occurs. Indeed, even if the nebulizer were to be inverted completely then all of the liquid would be retained within the reservoir 43 without any spillage out of the apertures 51.

From Figure 3 it would be seen in particular that even in the horizontal position liquid will be drawn up through the capillary 29 as the flange plate 13 extends down to the liquid.

It is well known that the three parts of the nebulizer must be separated for regular cleaning and removal of the flange plate 13 has been particularly difficult with the arrangement of the nebulizer of Figure 1. The increased diameter of the nebulizer and the provision of the fin 101 simplifies removal of the flange plate 13 since this can be more readily gripped for removal thereof.

The arrangement of Figure 8 is the same as Figure 2 except that the depth of the ring is 3mm instead of 1.5mm. Thus the lower most edge of the ring 102 is even further below the lower most point of the target 41 than in Figure 2.

In Figures 2 and 8, the ring 102, because it abuts the baffle plate 38 effectively forms a downwardly directed baffle or skirt.

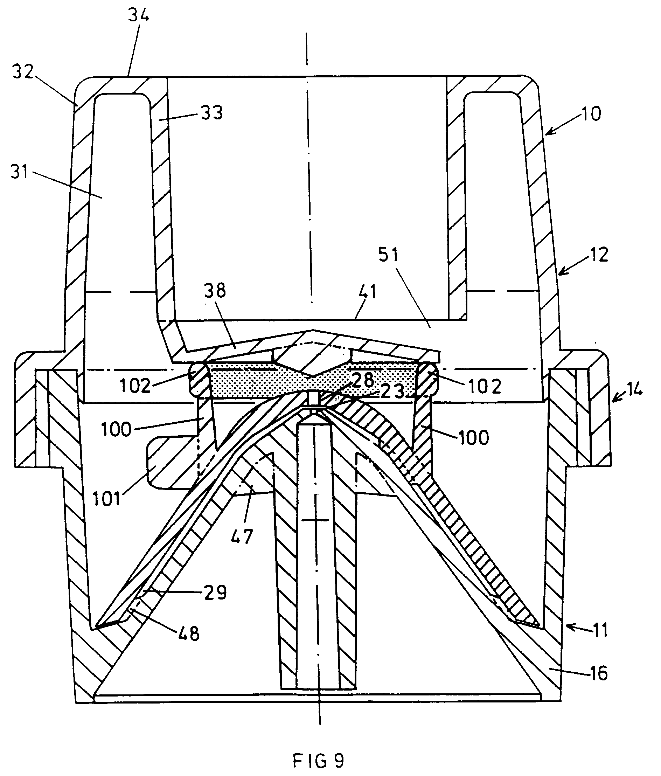

We also refer to Figure 9. This corresponds to Figure 2 except in place of the semispherical target 41 there is provided, a conical target 110. The angle of the surface of the conical target 110 with respect to its axis is between 55 degrees and 80 degrees, preferably 70 degrees.

As mentioned above, it is desirable to be able to produce nebulized droplets of a desired size range and a droplet size (i.e diameter) of less than 5 micron is desirable and for many purposes (although not all), a droplet size of less than 3.5 micron is particularly desirable and between 0.5 and 2 micron is particularly desirable.

Of course, in practice, there is a range of droplet sizes produced and it is desirable to be able to provide, for a particular nebulizer in use, a measure of the spread of droplet sizes and a mass median diameter (MMD).

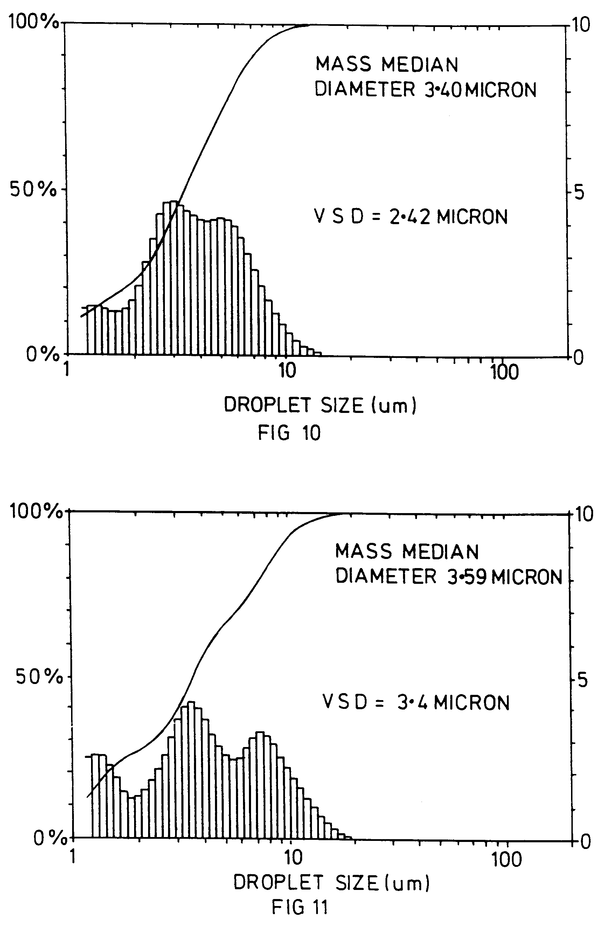

Figures 10 to 17 show test results analysing the droplet size distribution for a variety of nebulizers. These graphs have been produced by the use of a Malvern 2600C droplet size analyser manufactured by Malvern Instruments of Spring Lane South, Malvern, United Kingdom.

The Malvern 2600C is a laser diffraction device. To measure droplet size, a beam of laser radiation is shone through the droplets from the nebulizer. The smaller droplets diffract the laser light (i.e divert the laser light) at a larger angle and the larger droplets divert the light at a smaller angle. The light is focused by a lens on to a detector and the light intensity pattern on the detector is analysed by a computer. The computer then gives a volume distribution of variously sized droplets.

The nebulizer droplets cannot be counted to give a number distribution, because there are too many droplets for the computer to handle, and a number distribution would be uninformative. Importantly, a number distribution is misleading because a large number of nebulized small droplets can account for only a very small volume of drug solution nebulized, since volume is proportional to radius cubed.

A histogram of the volume distribution provides an easily discernable distribution of the droplet size against the quantity of drug.

The graphs in Figures 10 to 17, therefore, show volume distribution against droplet size for various nebulizers. They were all carried out under the same conditions, viz dry compressed air was passed through the nebulizer at 8 litres a minute and the nebuliser reservoir contained water.

The droplet size scale is a logarithmic scale extending between 1 and 100 micron. As can been seen the graph has two parts, a line graph and a histogram. The lefthand vertical scale relates to the line graph. The lefthand scale is of the percentage of the total volume of droplets below the relevant droplet size. The righthand scale which relates to the histogram is a scale of percentage of the total volume of droplets which are of a droplet size within the limits set by the width of the relevant bar of the histogram.

There is also provided with each graph a figure for the mass median diameter (MMD). This is the diameter above which 50% of the volume of droplets lie and below which a further half lies. This, combined with the Volume Standard Deviation gives an indication of the quality of the aerosol produced.

The VSD is a mathematical figure and is a good way of comparing the spread of various droplet distributions. If the VSD is large there is a large range of droplet sizes; if the VSD is small the particles are of similar sizes and the sizes are clustered around the MMD.

For the nebulizer to efficiently provide drug through to the alveoli of the lung, it is desirable that the mass median diameter (MMD) should be about 1.25 micron with a volume standard deviation of about 0.7 microns.

Figure 10 shows the droplet size distribution for a nebulizer in accordance with Figure 1. As can be seen, there are relatively few droplets in the range 1 - 2 micron, and there are a number of droplets going up beyond 10 micron. The mass median diameter (MMD) is 3.4 micron with a volume standard deviation of 2.42 microns. As a result few droplets will be of a size to reach the alveoli.

The applicants have obtained a nebulizer made in accordance with U.S. Patent No.4,588,129 and Figure 11 illustrates the droplet distribution of that nebulizer. As will be understood from that U.S. Patent specification No.4.588,129, there is some discussion of the value of the droplet size. It will be seen that the measured droplet distribution provides a volume distribution in which the bulk of droplets are approximately 3.5 micron in diameter, and the mean diameter is in fact measured to be 3.59 micron with a volume standard deviation of 3.4 microns.

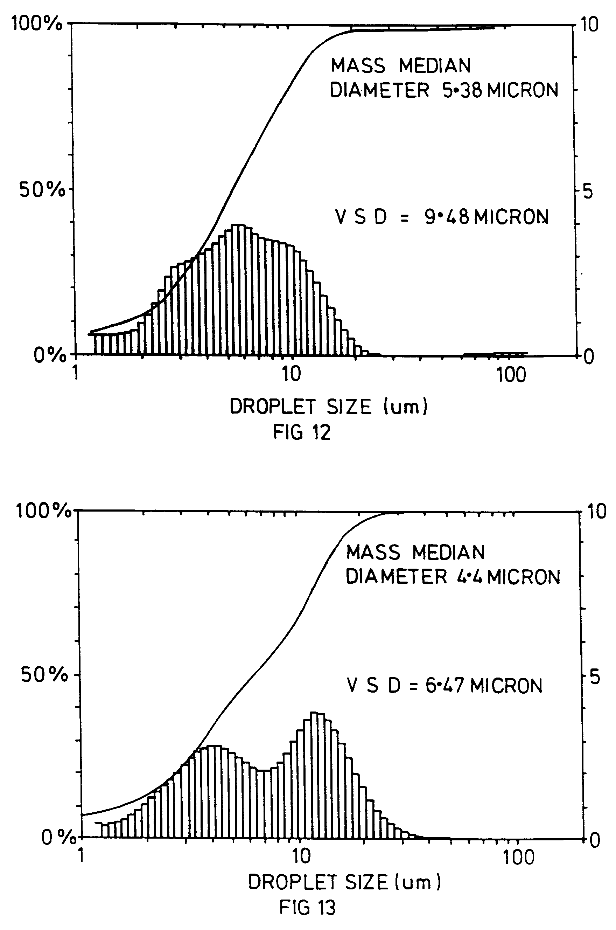

Figure 12 shows the droplet size distribution for a nebulizer made in accordance with U.S. Patent 4,746,067. A somewhat similar distribution is provided to that of Figure 11 but the mass median diameter (MMD) is greater at 5.38 micron with a volume standard deviation of 9.48 microns. This is a particularly high figure because there are some droplets in the 100 micron range. Once again, few droplets would reach the alveoli.

Figure 13 shows the droplet size distribution for a nebulizer in accordance with our European Patent Application 88300391.5. The mass median diameter (MMD) is 6.44 micron with a volume standard deviation of 6.47 micron.

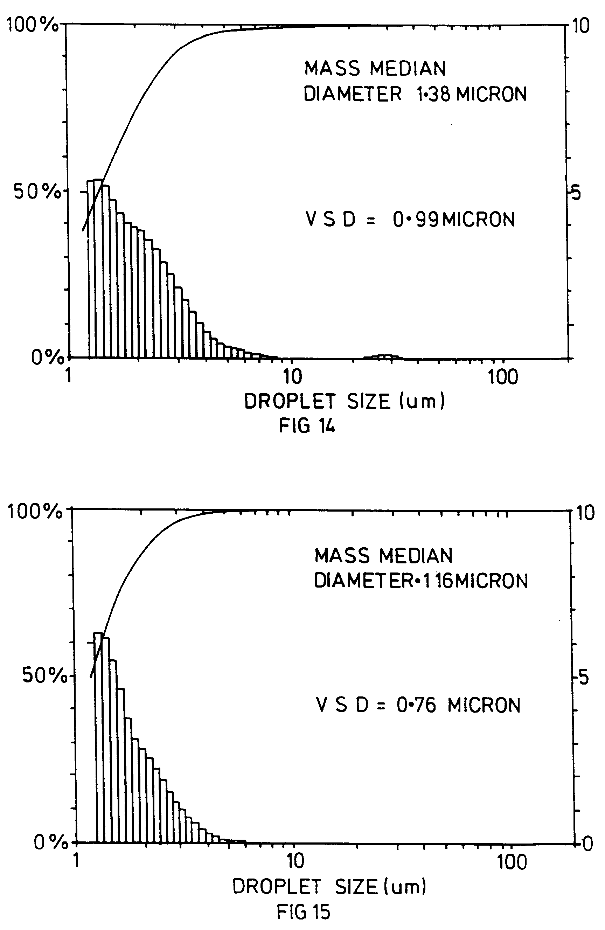

Figure 14 shows the droplet size distribution for a nebulizer of the type shown in Figure 2. It will be immediately seen that there is a dramatic improvement in the distribution, very many more droplets being of a smaller size and indeed virtually no droplets being of a size greater than about 8 micron. The mass median diameter (MMD) is 1.38 with a volume standard deviation of 0.99 micron. As a result a large proportion of the droplets would pass the alveoli.

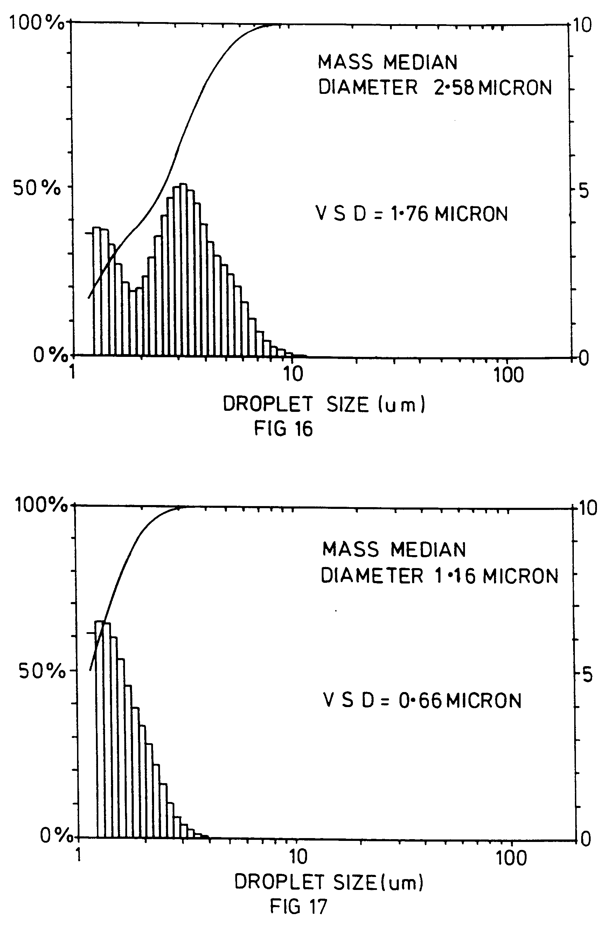

Figure 15 shows the droplet size distribution for the nebulizer of Figure 8, that is with a deeper ring. This shows a reasonable improvement upon the arrangement of Figure 14, the mass median diameter (MMD) now being 1.16 with a volume standard deviation of 0.76 micron, the improvement in the very small size droplets being particularly great. Once again, the bulk of the droplets would pass to the alveoli.

Figure 16 is a graph of the droplet size distribution of a nebulizer in accordance with Figure 2 but modified similar to that of U.S. Patent No.4,588,129, by having a rearwardly extending chamber between the target 41 and ring 100. It can be seen that this makes the situation considerably worse, reducing the number of small droplets considerably, and providing a peak at about 3 micron. As a consequence the mass median diameter (MMD) increases to 2.58 micron with a volume standard deviation of 1.76 micron.

Figure 17 is a graph of the droplet size distribution of a nebulizer according to Figure 9, that is with a conical target 41 . There is a very good droplet size distribution, generally corresponding to that of Figure 17, providing a large number of very small droplets and no droplets about 4 micron. The mass median diameter (MMD) is 1.16 micron and the volume standard deviations has dropped to 0.66 micron, a very good figure.

It will be seen therefore that the provision of the ring 102 dramatically improves the situation with regard to the droplet size distribution.

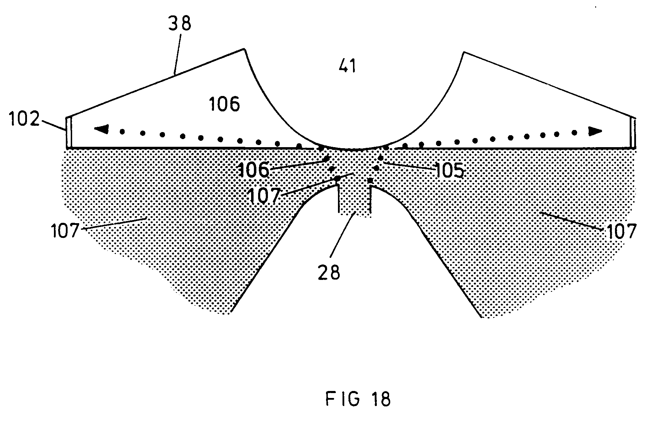

As a result of experimentation, we believe (although in no sense should the present invention be restricted as a result of this theory) that the gas/liquid jet 28 provides a cone 105 of nebulized gas/air mixture (see Figure 20), in which the periphery 106 of the cone 105 includes droplets of larger size. This conical jet 105 impinges upon the target 41 and in the arrangement of Figures 2 to 9, is then deflected and the ring 102 is disposed so as to collect the outer peripheral part 106 of the cone of nebulized gas/liquid (see Figure 18). As a result the smaller droplets (at 107) which are concentrated in the middle of the cone pass out of the nebulizer and the larger droplets are trapped.

As a result of considerable experimentation, we believe that a number of factors affect the size of the droplets provided by the nebulizer.

We also believe that the disposition of the lower edge of the ring, which effectively forms a skirt surrounding the target 41, is of importance. Indeed, we believe that the nebulizer works more effectively because the lower edge of the ring is below the lower most point of the target 41.

The provision of the relatively flat plate 38 extending between the target 41 and the ring 102 is clearly important. Thus the difference between the results shown in Figure 13 and that of Figure 15, wherein the only difference is that the relatively flat plate 38 has been replaced by a chamber as in U.S. Patent No.4,588,129 suggests that this relatively flat plate is important.

It will be understood from the above that it is possible to provide interchangeable parts to provide droplet size characteristics required. For example, by simply interchanging the flange plate 13 of the nebulizer of our European Patent specification 88300391.5 with the flange plate of Figure 2, one can change the characteristics of the nebulizer so as to produce a mass median diameter (MMD) which changes from 6.44 micron to 1.38 micron.

The invention is not restricted to the details of the foregoing examples.

## Claims

1. A nebulizer comprising:
a base part (11) including a base wall (17) extending to an apex;
a gas input (18) including an input connector (19);
a gas jet hole (23) extending through the base wall connecting with the interior of the input connector;
a gas/liquid output (39);
a flange plate (13) extending above said base wall over the gas jet hole, the under surface of said flange plate generally matching the upper surface of the base wall and being spaced from said base wall by a sufficiently small distance to provide a capillary path (29);
a reservoir (24) for liquid being provided by said base wall communicating with said capillary path; and,
a gas/liquid mixing jet (28) for mixing gas from the gas jet hole through the base wall with liquid from the capillary path, the gas/liquid mixing jet comprising a hole through the flange plate opposite and in axial alignment with said gas jet hole through the base wall;
an annular baffle plate (38), including a target (41) centrally mounted on said annular baffle plate opposite said gas/liquid mixing jet against which the gas/liquid jet formed by the gas/liquid mixing jet impinges to nebulize the liquid;
and,
outlet aperture means (51) adjacent the outer peripheral edge of said baffle plate and connecting with the gas/liquid output for allowing direct passage of the nebulized gas/liquid mixture from the nebulizer characterised by a ring member (102), in use, positioned so as to extend from the surface of the annular baffle plate, and being generally coaxial with the target, and in that, with the nebulizer in an upright orientation, the lowermost edge of the ring member (102) extends below the lowermost point of the target (41).

2. A nebulizer as claimed in claim 1 characterised in that the target is semispherical.

3. A nebulizer has claimed in claim 2 characterised in that the radius of the target is 2 - 5mm, preferably substantially 3.5mm.

4. A nebulizer as claimed in claim 3 characterised in that the radius of the target is substantially 2.5mm.

5. A nebulizer as claimed in any of claims 1 to 4 characterised in that the diameter of the ring means is 15 - 25mm.

6. A nebulizer as claimed in claim 5 characterised in that the diameter of the ring means is approximately 20mm diameter.

7. A nebulizer as claimed in any of claims 1 to 6 characterised in that the depth of the cross section of the ring means is 1 - 5mm.

8. A nebulizer as claimed in claim 7 characterised in that the depth of the cross section of the ring means is approximately 1.5mm.

9. A nebulizer as claimed in claim 7 characterised in that the depth of the cross section of the ring means is approximately 3mm.

10. A nebulizer as claimed in any of claims 1 to 9 characterised in that the target comprises a conical member.

11. A nebulizer as claimed in claim 10 characterised in that the angle of the surface of the conical target with respect to its axis is between 55 degrees and 80 degrees.

12. A nebulizer as claimed in claim 10 characterised in that the angle of the surface of the conical target with respect to its axis is approximately 70 degrees.

13. A nebulizer as claimed in any of claims 1 to 12 characterised in that the ring means is mounted by leg means (100), to the flange plate.

14. A nebulizer as claimed in any of claims 1 to 13 characterised in that the base part comprises an outer wall (16) and the base wall comprises a conical base wall (17) formed integrally with and extending smoothly and uninterruptedly from the outer wall to an apex, the outer surface of said base wall having a convex central portion including the apex.

15. A nebulizer as claimed in any of claims 1 to 14 characterised in that a wall of the reservoir itself forms the gas/liquid output for the nebulized gas and liquid mixture.

16. A nebulizer as claimed in any of claims 1 to 15 in which a cover (12) is provided for said reservoir, said cover incorporating said gas/liquid outlet.

17. A nebulizer as claimed in any of claims 1 to 16 characterised in that said reservoir includes a cover (12), said cover including said annular baffle plate.

18. A nebulizer as claimed in claim 1 characterised in that said nebulizer provides droplets of a mass median diameter (MMD) of less than 3.5 micron.

19. A nebulizer as claimed in claim 18, characterised in that the droplet has a mass median diameter (MMD) of less than approximately 1.5 micron.

## Patentansprüche

1. Zerstäubervorrichtung, umfassend
ein Basisteil (11) mit einer Basiswand (17), die sich bis zu einem Scheitel erstreckt;
einen Gaseinlaß (18) mit einem Einlaßanschluß (19);
eine Gasdüsenbohrung (23), die sich durch die Basiswand erstreckt und die an das Innere des Einlaßanschlußes angeschlossen ist;
einen Gas-Flüssigkeits-Auslaß (39);
eine Flanschplatte (13), die sich oberhalb der Basiswand über der Gasdüsenbohrung erstreckt, und deren untere Fläche im wesentlichen der oberen Fläche der Basiswand entspricht und gegenüber dieser um einen genügend kleinen Abstand versetzt ist, so daß ein Kapillarweg (29) geschaffen wird;
einen Vorratsraum (24) für die Flüssigkeit, definiert durch die Basiswand und mit dem Kapillarweg kommunizierend; und
eine Gas-Flüssigkeits-Mischdüse (28) zum Mischen von Gas aus der Gasdüsenbohrung durch die Basiswand mit Flüssigkeit vom Kapillarweg, wobei die Gas-Flüssigkeits-Mischdüse eine Bohrung in der Flanschplatte aufweist, die der Gasdüsenbohrung in der Basiswand gegenüberliegt und mit dieser fluchtet;
eine ringförmige Prallplatte (38), mit einem Zielkörper (41), der zentral an der ringförmigen Prallplatte gegenüber der Gas-Flüssigkeits-Mischdüse angeordnet ist, und gegen welche der Gas-Flüssigkeits-Strahlspalt, der seinerseits durch die Gas-Flüssigkeits-Mischdüse gebildet ist, um die Flüssigkeit zu zerstäuben; und
eine Auslaßöffnung (51), die im Bereich der äußeren Umfangskante der Prallplatte angeordnet ist und den Gas-Flüssigkeits-Auslaß leitend verbindet, um einen direkten Durchtritt des zerstäubten Gas-Flüssigkeits-Gemisches aus der Zerstäubervorrichtung zu ermöglichen mittels eines Ringelementes 102, das beim Gebrauch derart angeordnet ist, daß es sich von der Fläche der ringförmigen Prallplatte aus erstreckt und das im wesentlichen koaxial zum Zielkörper angeordnet ist;
dadurch gekennzeichnet, daß sich bei stehender Zerstäubervorrichtung die unterste Kante des Ringelementes 102 unterhalb des untersten Punktes des Zielkörpers (41) erstreckt.

2. Zerstäubervorrichtung nach Anspruch 1, dadurch gekennzeichnet, daß der Zielkörper halbkugelig ist.

3. Zerstäubervorrichtung nach Anspruch 2, dadurch gekennzeichnet, daß der Radius des Zerstäuberkörpers 2 bis 5 mm beträgt, am besten im wesentlichen 3,5 mm.

4. Zerstäubervorrichtung nach Anspruch 3, dadurch gekennzeichnet, daß der Radius des Zielkörpers im wesentlichen 2,5 mm beträgt.

5. Zerstäubervorrichtung nach einem der Ansprüche 1 bis 4, dadurch gekennzeichnet, daß der Durchmesser des Ringelementes 15 bis 25 mm beträgt.

6. Zerstäubervorrichtung nach Anspruch 5, dadurch gekennzeichnet, daß der Durchmesser des Ringelementes etwa 20 mm beträgt.

7. Zerstäubervorrichtung nach einem der Ansprüche 1 bis 6, dadurch gekennzeichnet, daß die Tiefe des Querschnittes des Ringelementes zwischen 1 und 5 mm liegt.

8. Zerstäubervorrichtung nach Anspruch 7, dadurch gekennzeichnet, daß die Tiefe des Querschnittes des Ringelementes etwa 1,5 mm beträgt.

9. Zerstäubervorrichtung nach Anspruch 7, dadurch gekennzeichnet, daß die Tiefe des Querschnittes des Ringelementes etwa 3 mm beträgt.

10. Zerstäubervorrichtung nach einem der Ansprüche 1 bis 9, dadurch gekennzeichnet, daß der Zielkörper ein konisches Element umfaßt.

11. Zerstäubervorrichtung nach Anspruch 10, dadurch gekennzeichnet, daß der Winkel der Fläche des konischen Zielkörpers in bezug auf seine Achse etwa 55 bis 80 Grad beträgt.

12. Zerstäubervorrichtung nach Anspruch 10, dadurch gekennzeichnet, daß der Winkel der Fläche des konischen Zielkörpers in bezug auf seine Achse annähernd 70 Grad beträgt.

13. Zerstäubervorrichtung nach einem der Ansprüche 1 bis 12, dadurch gekennzeichnet, daß das Ringelement durch Schenke 100 an der Flanschplatte montiert ist.

14. Zerstäubervorrichtung nach einem der Ansprüche 1 bis 13, dadurch gekennzeichnet, daß das Basisteil eine Außenwand (16) umfaßt, daß die Basiswand eine konische Basiswand (17) aufweist, die einteilig mit der Außenwand gebildet und sich ununterbrochen von der Außenwand zu einem Scheitel erstreckt, und daß die Außenfläche der Basiswand einen konvexen zentralen Bereich aufweist, eingeschlossen den Scheitel.

15. Zerstäubervorrichtung nach einem der Ansprüche 1 bis 14, dadurch gekennzeichnet, daß eine Wand des Vorratsraumes selbst den Gas-Flüssigkeits-Auslaß für zerstäubtes Gas-Flüssigkeits-Gemisch bildet.

16. Zerstäubervorrichtung nach einem der Ansprüche 1 bis 15, dadurch gekennzeichnet, daß ein Deckel (12) für den Vorratsraum vorgesehen ist, und den Gas-Flüssigkeits-Auslaß beinhaltet.

17. Zerstäubervorrichtung nach einem der Ansprüche 1 bis 16, dadurch gekennzeichnet, daß der Vorratsraum einen Deckel (12) aufweist, der die ringförmige Prallplatte beinhaltet.

18. Zerstäubervorrichtung nach Anspruch 1, dadurch gekennzeichnet, daß sie Tröpfchen eines Mittleren Massendurchmessers (MMD) von weniger als 3,5 Mikron erzeugt.

19. Zerstäubervorrichtung nach Anspruch 18, dadurch gekennzeichnet, daß das Tröpfchen einen Mittleren Massendurchmesser (MMD) von weniger als annähernd 1,5 Mikron aufweist.

## Revendications

1. Nébuliseur comprenant :
une partie de base (1) comprenant une parni de base (17) s'étendant vers un sommet ;
une entrée de gaz (18) comprenant un connecteur d'entrée (19) ;
un trou à jet de gaz (23) traversant la paroi de base en communication avec l'intérieur du connecteur d'entrée;
une sortie de gaz/liquide (39) ;
une plaque de bride (13) s'étendant au-dessus de ladite paroi de base au-delà du trou à jet de gaz, la surface intérieure de ladite plaque de bride s'adaptant de façon générale à la surface supérieure de la paroi de base et étant éloignée de ladite paroi de base d'une distance suffisamment petite pour fournir un passage capillaire (29) ;
un réservoir (24) à liquide étant fourni par ladite paroi de base communiquant avec ledit passage capillaire ; et
une buse mélangeuse de gaz/liquide (28) pour mélanger du gaz provenant du trou à jet de gaz traversant la paroi de base avec du liquide provenant du passage capillaire , la buse mélangeuse de gaz/liquide comprenant un trou traversant la plaque de bride opposée et en alignement axial avec ledit trou à jet de gaz traversant la paroi de base ;
une plaque déflectrice annulaire (38), comprenant une cible (41) montée de façon centrale sur ladite plaque déflectrice annulaire en face de ladite buse mélangeuse de gaz/liquide contre laquelle vient frapper le jet de gaz/liquide formé par la buse mélangeuse de gaz/liquide pour nébuliser le liquide ; et
un moyen d'orifice de sortie (51) adjacent à la bordure périphérique extérieure de ladite plaque déflectrice et communiquant avec la sortie de gaz/liquide pour permettre un passage direct du mélange de gaz/liquide nébulisé issu du nébuliseur, caractérisé par un élément annulaire (102), situé, en fonctionnement, de façon à s'étendre à partir de la surface de la plaque déflectrice annulaire, et étant généralement coaxial à la cible, et en ce que, le nébuliseur se trouvant dans une orientation verticale, la bordure la plus basse de l'élément annulaire (102) s'étend au-dessous du point le plus bas de la cible (41).

2. Nébuliseur selon la revendication 1, caractérisé en ce que la cible est semi-sphérique.

3. Nébuliseur selon la revendication 2, caractérisé en ce que le rayon de la cible est de 2 à 5 mm, de préférence très proche de 3,5 mm.

4. Nébuliseur selon la revendication 3, caractérisé en ce que le rayon de la cible est sensiblement de 2,5 mm.

5. Nébuliseur selon l'une quelconque des revendications 1 à 4, caractérisé en ce que le diamètre de l'élément annulaire est de 15 à 25 mm.

6. Nébuliseur selon la revendication 5, caractérisé en ce que le diamètre de l'élément annulaire est approximativement un diamètre de 20 mm.

7. Nébuliseur selon l'une quelconque des revendications 1 à 6, caractérisé en ce que l'épaisseur de la section transversale de l'élément annulaire est de 1 à 5 mm.

8. Nébuliseur selon la revendication 7, caractérisé en ce que l'épaisseur de la section transversale de l'élément annulaire est d'environ 1,5 mm.

9. Nébuliseur selon la revendication 7 , caractérisé en ce que l'épaisseur de la section transversale de l'élément annulaire est approximativement de 3 mm.

10. Nébuliseur selon l'une quelconque des revendications 1 à 9, caractérisé en ce que la cible comprend un élément conique.

11. Nébuliseur selon la revendication 10, caractérisé en ce que l'angle que fait la surface de la cible conique avec son axe se situe entre 55 degrés et 80 degrés.

12. Nébuliseur selon la revendication 10, caractérisé en ce que l'angle que fait la surface de la cible conique avec son axe est approximativement de 70 degrés .

13. Nébuliseur selon l'une quelconque des revendications 1 à 12, caractérisé en ce que l'élément annulaire est monté sur la plaque de bride à l'aide de moyens de bras (100).

14. Nébuliseur selon l'une quelconque des revendications 1 à 13, caractérisé en ce que la partie de base comprend une paroi extérieure (16) et la paroi de base comprend une paroi de base conique (17) formée de façon intégrée à la paroi extérieure et s'étendant de façon régulière et non interrompue à partir de la paroi extérieure vers un sommet, la surface extérieure de ladite paroi de base comportant une partie centrale convexe incluant le sommet.

15. Nébuliseur selon l'une quelconque des revendications 1 à 14, caractérisé en ce qu' une paroi du réservoir lui-même forme la sortie de gaz/liquide pour le mélange de gaz et de liquide nébulisé.

16. Nébuliseur selon l'une quelconque des revendications 1 à 15, dans lequel un chapeau (12) est prévu pour ledit réservoir, ledit chapeau incorporant ladite sortie de gaz/liquide.

17. Nébuliseur selon l'une quelconque des revendications 1 à 16, caractérisé en ce que ledit réservoir comprend un chapeau (12), ledit chapeau incluant ladite plaque déflectrice annulaire.

18. Nébuliseur selon la revendication 1, caractérisé en ce que ledit nébuliseur fournit des gouttelettes d'un diamètre médian en volume (MMD) inférieur à 3,5 microns.

19. Nébuliseur selon la revendication 18, caractérisé en ce que la gouttelette possède un diamètre médian en volume (MMD) inférieur à environ 1,5 micron.
